(19) Europäisches Patentamt / European Patent Office / Office européen des brevets

(11) **EP 1 927 339 A2**

(12) **DEMANDE DE BREVET EUROPEEN**

(43) Date de publication:
**04.06.2008 Bulletin 2008/23**

(51) Int Cl.:
*A61K 8/25* *(2006.01)*     *A61K 8/29* *(2006.01)*
*A61Q 1/04* *(2006.01)*     *A61Q 1/06* *(2006.01)*
*A61Q 3/02* *(2006.01)*

(21) Numéro de dépôt: **07120906.8**

(22) Date de dépôt: **16.11.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité:
17.11.2006   FR 0654978
17.11.2006   FR 0654977
17.11.2006   FR 0654975
17.11.2006   FR 0654981
12.12.2006   FR 0655456
12.12.2006   FR 0655454
12.12.2006   FR 0655452
12.12.2006   FR 0655460

(71) Demandeur: **L'ORÉAL**
**75008 Paris (FR)**

(72) Inventeur: **Thevenet, Ludovic**
**92340, BOURG LA REINE (FR)**

(74) Mandataire: **Tanty, François**
**Nony & Associés,**
**3, rue de Penthièvre**
**75008 Paris (FR)**

(54) **Composition cosmétique couvrante**

(57) La présente invention concerne une composition cosmétique de couvrance supérieure ou égale à 25, mieux 30, comportant, dans un milieu cosmétiquement acceptable aqueux, au moins un pigment interférentiel multicouche en une teneur conférant à la composition une variation de couleur ΔE à l'application supérieure ou égale à 5.

Fig.2

EP 1 927 339 A2

## Description

La présente invention concerne les compositions cosmétiques, et plus particulièrement celles destinées au maquillage des matières kératiniques.

## Arrière-plan

Il est connu d'introduire dans les compositions de maquillage des pigments diffusants pour produire des couleurs par un phénomène d'absorption de la lumière par des chromophores spécifiques.

A ces pigments diffusants, qui sont nécessaires pour bénéficier d'un fond coloré continu et suffisamment couvrant, peuvent être ajoutées des particules à effet(s) pour créer par exemple des points de brillance ou conférer un aspect nacré.

Toutefois, l'intensité de la couleur produite par ces compositions peut s'avérer insuffisante pour conduire à un résultat complètement satisfaisant.

Les pigments interférentiels multicouche, comportant un empilement de couches dont les indices de réfraction et les épaisseurs sont convenablement choisis afin de générer une couleur par un phénomène d'interférences, permettent de produire une intensité de couleur plus importante que les pigments diffusants ci-dessus.

A la connaissance de l'inventeur, dans les compositions disponibles commercialement, à l'exception des poudres, ces pigments interférentiels multicouche sont employés à une concentration massique n'excédant pas 5 %.

Il est connu par ailleurs par les fards à paupières de marque CHRYSALIDE de la société LANCÔME de conférer à la fois couvrance et intensité colorielle au moyen d'un maquillage faisant intervenir un premier geste consistant à déposer sur les matières kératiniques une couche de base de couleur noire contenant un pigment diffusant apportant de la couvrance et de déposer ensuite par un second geste sur cette couche de base une composition apportant de la couleur par le biais d'un pigment interférentiel multicouche. Sans la couche de base, la couche de recouvrement est pratiquement invisible car non couvrante et sans couleur.

Le recours à deux applications successives complique le maquillage et rend le conditionnement plus coûteux.

## Résumé

Composition couvrante

Il existe un besoin pour bénéficier d'une composition capable d'apporter de la couvrance et de produire une couleur relativement saturée, afin de pouvoir obtenir en un seul geste un maquillage à la fois couvrant et coloré.

Par ailleurs, il est souhaitable de bénéficier de compositions cosmétiques destinées au maquillage des matières kératiniques qui présentent de nouveaux effets susceptibles d'attirer les consommateurs, sans que ces nouveaux effets ne se produisent au détriment de la qualité du maquillage obtenu.

L'invention vise notamment à répondre à tout ou partie des besoins identifiés ci-dessus.

La présente invention a pour objet, selon l'un de ses aspects, une composition cosmétique de couvrance supérieure ou égale à 25, mieux 30, comportant, dans un milieu cosmétiquement acceptable aqueux, au moins un pigment interférentiel multicouche en une teneur conférant à la composition une variation de couleur $\Delta E$ à l'application supérieure ou égale à 5.

L'invention a également pour objet, selon un autre de ses aspects, une composition cosmétique de couvrance supérieure ou égale à 25, mieux 30, comportant, dans un milieu cosmétiquement acceptable anhydre, au moins un pigment interférentiel multicouche, et en une teneur conférant à la composition une variation de couleur $\Delta E$ de la composition à l'application supérieure ou égale à 5.

La couleur après application est déterminée pour les besoins du calcul de $\Delta E$ après étalement de la composition sur une carte de contraste, comme pour la mesure de la couvrance.

L'invention a encore pour objet, selon un autre de ses aspects, une composition cosmétique de couvrance supérieure ou égale à 25, mieux 30, comportant, dans un milieu cosmétiquement acceptable, au moins un pigment interférentiel multicouche en une teneur conférant à la composition une variation de couleur $\Delta E$ à l'application supérieure ou égale à 5, le pigment interférentiel multicouche produisant une couleur de longueur d'onde dominante hors de l'intervalle allant de 580 nm à 650 nm.

La couvrance de la composition peut être due, le cas échéant, seulement à la teneur en pigment(s) interférentiel (s) multicouche.

L'invention a encore pour objet, selon un autre de ses aspects, une composition cosmétique de couvrance supérieure ou égale à 25, mieux 30, comportant, dans un milieu cosmétiquement acceptable, au moins un pigment interférentiel multicouche en une teneur conférant à la composition une variation de couleur $\Delta E$ à l'application supérieure ou égale à 5, la couleur de la composition dans la masse étant blanche.

Selon les compositions, l'écart $\Delta E$ peut être compris par exemple entre 5 et 30, notamment supérieur à toute valeur entière comprise dans cet intervalle.

La couvrance peut être comprise entre 30 et 70, notamment supérieure à toute valeur entière comprise dans cet intervalle, par exemple supérieure ou égale à 40.

Dans les pigments interférentiels multicouche, la production de la couleur par le phénomène d'interférences est en compétition avec la production de la couleur par le phénomène d'absorption par la couche de surface du pigment.

Ainsi, lorsque la concentration en pigment augmente suffisamment, la couleur produite par le phénomène d'interférences diminue au profit de celle produite par absorption.

L'invention, en exploitant cette propriété, permet d'observer une variation de couleur de la composition à l'application, qui confère un aspect ludique à l'utilisation.

L'invention offre également de nouvelles possibilités en matière de commercialisation des compositions cosmétiques, en permettant de jouer au niveau du conditionnement sur cette variation de couleur avant et après application.

La teneur massique en pigment interférentiel multicouche peut aller de 7 à 20 %, mieux 8 à 15 %, notamment dans le cas d'une composition non pulvérulente, par exemple liquide ou coulée.

Pour une composition pulvérulente, libre ou compacte, la teneur en pigment interférentiel multicouche va par exemple de 40 à 95 %, mieux 50 à 80 %.

La couleur de la composition dans la masse peut être blanche, c'est-à-dire achromatique au sens de la CIE.

La composition, en masse, peut avoir un indice de blancheur supérieur ou égal à 40.

La composition peut ne pas comporter d'autre agent de coloration que le ou les pigments interférentiels multicouche.

Le pigment interférentiel multicouche peut comporter au moins quatre couches, par exemple.

Le pigment interférentiel multicouche peut comporter un substrat en une matière plus ou moins rugueuse, ce qui peut permettre de conférer plus ou moins de brillance à la composition.

Le substrat est par exemple choisi en mica naturel, relativement rugueux, en mica synthétique, en alumine, silice ou en verre ou métal pour une surface plus lisse.

Dans des exemples, la composition comporte plus de 30 % en masse au total d'huile(s).

Dans des exemples, la composition comporte plus de 10 % en masse au total de cire(s).

La composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

L'invention a encore pour objet, selon un autre de ses aspects, une composition cosmétique non pulvérulente, par exemple liquide (à 25 °C) ou coulée, comportant, dans un milieu cosmétiquement acceptable :

- entre 7 et 20 %, mieux 8 à 15 % en masse, au total, d'un ou plusieurs pigments interférentiels multicouche ayant un substrat minéral, par exemple en silice, mica, alumine, verre ou métal.

L'invention a encore pour objet, selon un autre de ses aspects, une composition cosmétique pulvérulente comportant, dans un milieu cosmétiquement acceptable :

- entre 40 et 95 %, mieux 40 et 80 % en masse, au total, d'un ou plusieurs pigments interférentiels multicouche ayant un substrat minéral, par exemple en silice, mica, alumine verre ou métal.

L'invention a encore pour objet, selon un autre de ses aspects, un ensemble de conditionnement comportant :

- une composition telle que définie ci-dessus,
- un moyen permettant de renseigner l'utilisateur sur la couleur de la composition après application sur des matières kératiniques (lèvres, peau, cils, sourcils, cheveux, ongles). Il peut s'agir par exemple d'une impression d'une encre ou d'un vernis, d'une couche mince de composition ou d'un moulage ou surmoulage d'un matériau incorporant le ou les pigments interférentiels multicouche.

L'invention a encore pour objet, indépendamment ou en combinaison avec ce qui précède, une composition cosmétique de couvrance supérieure ou égale à 25 comportant, dans un milieu cosmétiquement acceptable, au moins un pigment interférentiel multicouche non opaque apte à conférer une couleur blanche dans la masse.

Le mot « opaque » au sens de l'invention signifie ne laissant passer aucune lumière. Par opposition, le terme « non opaque » ou transparent signifie « qui laisse passer au moins un peu la lumière ».

Composition blanche dans la masse

L'invention a encore pour objet, selon l'un de ses aspects, indépendamment ou en combinaison avec ce qui précède, une composition cosmétique solide, notamment en stick, comportant, dans un milieu cosmétiquement acceptable, au moins un pigment interférentiel multicouche apte à conférer à la composition une couleur blanche dans la masse, et à provoquer après application une variation de couleur ΔE de la composition d'au moins 5.

Par « stick », encore appelé parfois « raisin » ou « bâton », on désigne une composition sous forme solide, généralement sous forme de bloc allongé, permettant un transfert sur les matières à maquiller par friction. Un stick peut être obtenu par exemple par moulage ou extrusion.

La couleur après application est déterminée pour les besoins du calcul de ΔE après étalement de la composition sur une carte de contraste, comme pour la mesure de la couvrance. La composition, en masse, peut avoir un indice de blancheur supérieur ou égal à 40. La composition peut être anhydre ou aqueuse.

Selon les compositions, l'écart ΔE peut être par exemple compris entre 5 et 30, notamment être supérieur à toute valeur entière comprise dans cet intervalle.

La couvrance de la composition peut être comprise entre 30 et 70, notamment être supérieure à toute valeur entière comprise dans cet intervalle, par exemple supérieure ou égale à 40.

La teneur massique en pigment interférentiel multicouche peut aller de 7 à 20 %, mieux 8 à 15 %, notamment dans le cas d'une composition non pulvérulente, par exemple liquide ou en stick.

Pour une composition pulvérulente, libre ou compactée, la teneur en pigment interférentiel multicouche va par exemple de 40 à 95 %, mieux 50 à 80 %.

La composition peut ne pas comporter d'autre agent de coloration que le ou les pigments interférentiels multicouche.

Le pigment interférentiel multicouche peut comporter au moins quatre couches, par exemple. Le pigment interférentiel multicouche peut comporter un substrat en une matière plus ou moins rugueuse, ce qui peut permettre de conférer plus ou moins de brillance à la composition. Le substrat est par exemple choisi en mica naturel, relativement rugueux, en mica synthétique, en alumine, silice ou en verre ou métal pour une surface plus lisse.

La composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

L'invention a encore pour objet, selon un autre de ses aspects, indépendamment ou en combinaison avec ce qui précède, une composition cosmétique comportant, dans un milieu cosmétiquement acceptable, au moins un pigment interférentiel multicouche apte à conférer à la composition une couleur blanche dans la masse, d'indice de blancheur supérieur ou égal à 40, la teneur massique total en pigment interférentiel multicouche allant de 7 à 20 % dans le cas d'une composition non pulvérulente et de 40 à 95 %, mieux 40 à 80%, dans le cas d'une composition pulvérulente.

L'invention a encore pour objet, selon un autre de ses aspects, indépendamment ou en combinaison avec ce qui précède, un ensemble de conditionnement comportant:

- une composition blanche dans la masse telle que définie ci-dessus,
- un moyen permettant de renseigner l'utilisateur sur la couleur de la composition après application sur des matières kératiniques (peau, lèvres, cheveux, ongles cils, sourcils). Il peut s'agir par exemple d'une impression d'une encre ou d'un vernis, d'une couche mince de composition ou d'un moulage ou surmoulage d'un matériau incorporant le ou les pigments interférentiels multicouche.

Ensemble pour application bi-couche

L'invention a encore pour objet, selon l'un de ses aspects, indépendamment ou en combinaison avec ce qui précède, un ensemble comportant :

- une première composition cosmétique à appliquer sur des matières kératiniques, comportant dans un milieu cosmétiquement acceptable au moins un pigment interférentiel multicouche, la première composition présentant une couvrance supérieure ou égale à 25, mieux 30, la teneur en pigment interférentiel multicouche conférant à la composition un changement de couleur ΔE d'au moins 2, mieux 5 entre la couleur dans la masse et celle après application sur les matières kératiniques,
- une deuxième composition appelée composition de recouvrement ou *top coat,* à appliquer sur la première, ou
- une deuxième composition appelée composition de base ou *base coat,* à appliquer avant la première sur les matières kératiniques.

La deuxième composition est de préférence liquide.

La deuxième composition peut comporter une phase grasse, afin de conférer de la brillance au maquillage.

La deuxième composition peut être transparente, afin de ne pas affecter la saturation de la couleur produite par la première composition.

La deuxième composition peut être dépourvue de corps solides, afin de ne pas diffuser la lumière réfléchie par la première composition. En variante, la deuxième composition peut comporter au moins un pigment à effet, notamment un pigment réfléchissant métallique des pigments interférentiels, des composés à propriétés thermochromiques, photochromiques, tribochromiques, piézochromes, solvatochromes ou mécano luminescentes.

La première composition peut être anhydre ou aqueuse.

Selon les premières compositions, l'écart ΔE peut être supérieur à toute valeur entière comprise dans l'intervalle 5 à 30.

La couvrance de la première composition ou de la composition de base peut être supérieure à 30, notamment être supérieure à toute valeur entière comprise dans l'intervalle 30 à 70, par exemple supérieure ou égale à 40.

La teneur massique en pigment interférentiel multicouche dans la première composition peut aller de 7 à 15 %, mieux 8 à 15 %, notamment dans le cas d'une première composition non pulvérulente, par exemple liquide ou en stick.

Pour une première composition pulvérulente, la teneur en pigment interférentiel multicouche va par exemple de 40 à 95 %, mieux 40 à 80 %.

La couleur de la première composition dans la masse peut être blanche. La composition de base, en masse, peut avoir un indice de blancheur supérieur ou égal à 40.

La première composition peut ne pas comporter d'autre agent de coloration que le ou les pigments interférentiels multicouche.

La première composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

L'ensemble peut comporter un moyen pour indiquer la couleur après application de la première composition ou après application des deux compositions de l'ensemble, sur les matières kératiniques (peau, lèvres, ongles, cils, sourcils, cheveux).

Il peut s'agir par exemple d'une impression d'une encre ou d'un vernis, d'une couche mince de composition ou d'un moulage ou surmoulage d'un matériau incorporant le ou les pigments interférentiels multicouche.

La deuxième composition de recouvrement comporte un milieu cosmétiquement acceptable.

La formulation de ce milieu est choisie de façon à permettre l'application sur la première composition, afin par exemple de conférer de la brillance et/ou améliorer la tenue et/ou nuancer un effet optique apporté par la première composition.

La deuxième composition de recouvrement peut comporter un corps gras liquide ou un agent filmogène.

La deuxième composition de recouvrement peut comporter un colorant et un ou plusieurs actifs et autres composés.

La deuxième composition de recouvrement peut présenter toute forme galénique compatible avec l'application sur la première composition.

Lorsque l'obtention de brillance est recherchée, la composition de recouvrement est de préférence liquide et transparente, comportant avantageusement une phase grasse.

La deuxième composition peut comporter au moins un pigment à effet. Ce pigment sera dans une teneur qui n'affecte pas l'observation de la couleur produite par la composition de base une fois appliquée sur les matières kératiniques.

Chaque composition peut être conditionnée dans tout réceptacle ou sur tout support prévu à cet effet. Les première et deuxième compositions peuvent être contenues, le cas échéant, dans deux compartiments d'un même dispositif de conditionnement et/ou dans un même emballage avant la première utilisation.

Chaque composition peut être appliquée en utilisant un applicateur, floqué ou non, par exemple une mousse, un embout, un pinceau, un feutre, une spatule, un fritté, une brosse, un peigne, un tissé ou non tissé.

L'application peut encore s'effectuer avec le doigt ou en déposant directement chaque composition sur le support à maquiller, par exemple par friction dans le cas d'un stick ou par pulvérisation ou projection à l'aide d'un dispositif piézoélectrique ou électrostatique ou par transfert d'une couche de composition préalablement déposée sur un support intermédiaire.

La première composition peut être conditionnée dans un dispositif de conditionnement qui laisse apparaître la couleur de la composition dans la masse.

Il peut s'agir d'un récipient ayant un corps au moins partiellement transparent et/ou d'un dispositif de conditionnement comportant un organe de bouchage au moins partiellement transparent.

La première composition peut être conditionnée dans un dispositif qui permet de voir à la fois la couleur de la composition dans la masse et la couleur de la composition après application sur des matières kératiniques.

La première composition peut encore être conditionnée dans un dispositif qui comporte des moyens représentatifs de la couleur révélée à l'application, par exemple un dépôt d'une couche de la première composition ou d'une encre ou vernis comportant les mêmes agents de coloration que la première composition.

Le cas échéant, la première composition ou la deuxième composition de recouvrement peut être conditionnée avec un aimant permettant de modifier l'orientation des particules de pigment interférentiel multicouche, lorsque ce pigment présente une susceptibilité magnétique non nulle.

Stick multicolore

L'invention a encore pour objet, selon l'un de ses aspects, indépendamment ou en combinaison avec ce qui précède, un produit solide, comportant au moins :

- un premier bloc d'une première composition cosmétique contenant, dans un milieu cosmétiquement acceptable, au moins un premier pigment interférentiel multicouche,
- un deuxième bloc d'une deuxième composition cosmétique, contenant dans un milieu cosmétiquement acceptable au moins un deuxième pigment interférentiel multicouche différent du premier,
  l'une au moins des première et deuxième compositions étant apte à présenter une première couleur dans la masse et une deuxième couleur après application, les première et deuxième couleurs différant d'un écart ΔE supérieur ou égal à 2, mieux 5.

Chacune des première et deuxième compositions peut être apte à présenter une première couleur dans la masse et une deuxième couleur, après application sur des matières kératiniques, les première et deuxième couleurs de chaque composition différant d'un écart ΔE supérieur ou égal à 5.

Les premier et deuxième blocs peuvent s'étendre sur toute la longueur d'un stick (encore appelé parfois raisin ou bâtonnet) ou sur toute l'épaisseur du produit lorsque celui-ci est coulé ou compacté dans une coupelle.

Les premier et deuxième blocs peuvent être concentriques ou disposés autrement.

Selon les compositions, l'écart ΔE peut être compris par exemple entre 5 et 30.

La couvrance de l'une des compositions peut être supérieure à 25, par exemple comprise entre 30 et 70. La couvrance de chaque composition peut être supérieure ou égale à 25, mieux 30. De préférence, la couvrance de chaque composition est sensiblement la même. La couvrance du produit fondu puis étalé avec mélange des compositions des différents blocs peut être supérieure ou égale à 25, par exemple comprise entre 30 et 70.

Selon cet aspect, l'invention offre un moyen de surprendre le consommateur en apportant la possibilité de révéler plusieurs couleurs à l'application, dans certains cas.

La teneur massique en pigment interférentiel multicouche dans l'une au moins des compositions peut aller de 7 à 20 %, mieux 8 à 15 %.

La couleur de l'une au moins des compositions dans la masse peut être blanche. Tous les blocs peuvent être blancs, c'est-à-dire achromatique au sens de la CIE, d'un indice de blancheur supérieur ou égal à 40 par exemple.

L'une au moins des compositions peut ne pas comporter d'autre agent de coloration que le ou les pigments interférentiels multicouche. Ce peut être le cas pour toutes les compositions du produit.

Les compositions du produit peuvent comporter deux pigments interférentiels multicouche respectifs ayant des couches constituées des mêmes matériaux.

Au moins une couche d'un pigment de l'une des compositions peut avoir une épaisseur différente d'une couche correspondante d'un pigment de l'autre composition, de façon à produire des couleurs différentes.

L'invention a encore pour objet, selon un autre de ses aspects, indépendamment ou en combinaison avec ce qui précède, un ensemble de conditionnement comportant :

- un produit solide tel que défini ci-dessus,
- un moyen permettant de renseigner l'utilisateur sur la couleur de l'une au moins des compositions du produit après application, voire d'une couleur formée par la superposition des compositions à l'application. Il peut s'agir par exemple d'une impression d'une encre ou d'un vernis, d'une couche mince de composition ou d'un moulage ou surmoulage d'un matériau incorporant le ou les pigments interférentiels multicouche.

Au sens de la présente invention, on entend englober par « produit solide » un produit ayant la propriété d'être dénué de la faculté d'écoulement sous l'action de son propre poids, dans des conditions normales de stockage.

Un produit solide peut être un produit dont la viscosité n'est pas mesurable.

Le produit solide peut, le cas échéant, présenter un aspect pâteux à température ambiante (25 °C).

L'un des blocs au moins pourra présenter un point de fusion ou une température de transition thermique (par exemple un point de ramollissement) supérieure à 25 °C, notamment pouvant varier de 25 à 85 °C, voire de 30 à 60 °C et en particulier de 30 à 45 °C.

La dureté de l'un au moins des blocs de composition pourra varier par exemple de 0,001 à 0,5MPa, notamment de 0,005 à 0,4MPa.

La dureté d'un bloc peut être déterminée par la mesure de la force en compression à 20 °C à l'aide du texturomètre vendu sous la dénomination TA-XT2i® par la société RHEO, équipé d'un cylindre en inox d'un diamètre de 2 mm se déplaçant à la vitesse de mesure de 0,1 mm/s, et pénétrant dans la composition de ce bloc à une profondeur de pénétration de 0,3 mm.

La valeur de la dureté est la force de compression maximale mesurée divisée par la surface du cylindre du texturomètre en contact avec la composition.

Dans le cas particulier des rouges à lèvres, la dureté peut également être mesurée par la méthode dite du fil à couper le beurre, qui consiste à couper un stick de rouge à lèvres de 8,1 mm de diamètre et à mesurer la dureté à 20 °C, au moyen d'un dynamomètre DFGHS 2 de la société Indelco-Chatillon se déplaçant à une vitesse de 100 mm/minute.

La dureté mesurée est exprimée comme la force de cisaillement (exprimée en grammeforce) nécessaire pour couper le stick dans ces conditions. Selon cette méthode, la dureté d'un produit solide selon l'invention va par exemple de 50 à 300 g, par exemple de 100 à 250 g et par exemple de 120 à 230 g.

**Mesure de la couvrance**

Compositions liquides (à 25°C)

Par « composition liquide », on entend une composition dont on peut mesurer la viscosité. Une composition liquide peut s'écouler sous l'effet de son propre poids.

La couvrance des compositions est mesurée à épaisseur finie de 50 $\mu$m, les compositions liquides étant par exemple des compositions à appliquer sur les lèvres, notamment des rouges à lèvres liquides, brillants à lèvres liquides et baumes à lèvres liquides, des vernis à ongles, des fards à paupières, des fonds de teint liquides, des mascaras et autres produits de maquillage liquides non destinés à être à appliqués sur les lèvres.

La composition est étalée sur des cartes de contraste noir mat et blanc mat, par exemple de marque LENETA Form WP1 pour la carte noir mat et Leneta 1A pour la carte blanc mat.

L'application peut s'effectuer avec un étaleur automatique.

Les mesures s'effectuent sur les compositions ainsi étalées.

Compositions solides (à 25°C)

Les compositions solides sont celles dont on ne peut mesurer la viscosité.

Il peut s'agir de compositions coulées en stick ou pulvérulentes, sous forme de poudres libres ou compactées.

a) Pour les compositions solides pulvérulentes, libres ou compactées, la composition est appliquée en utilisant les mêmes cartes de contraste que ci-dessus, recouvertes d'un ruban adhésif transparent, légèrement rugueux, par exemple de marque BLENDERM® de la société 3M et de référence 15025, collé par la face adhésive sur les cartes de contraste.

La composition est déposée sur le ruban adhésif de manière à obtenir un dépôt homogène de 0,5 mg/cm$^2$ $\pm$ 0,02 mg/cm$^2$.

Il est possible d'utiliser pour effectuer le dépôt une éponge chargée avec la composition et montée sur un appareil de délitage qui fait effectuer des mouvements prédéfinis à l'éponge. L'éponge est par exemple une éponge à usage unique de type « LANCÔME - Photogenic », utilisée du coté rose.

b) Les compositions en stick sont fondues, par exemple à 90°C, puis étalées avec une épaisseur de 50 $\mu$m à l'état liquide sur des cartes de contraste noir mat et blanc mat, par exemple de mêmes références que ci-dessus, non recouvertes de BLENDERM®. Le barreau d'étalement est maintenu à la même température que la composition, de façon à éviter un choc thermique.

Mesures et calculs

Des spectres de réflectance sont acquis à l'aide d'un spectrocolorimètre MINOLTA 3700-d (géométrie de mesure diffuse/8° et observation D65/10°, mode composante spéculaire exclue, petite ouverture (CREISS)) sur les fonds noir et blanc, les cartes de contraste étant éventuellement recouvertes de BLENDERM® comme indiqué ci-dessus.

Les spectres sont exprimés en coordonnées colorimétriques dans l'espace CIELab76 au sens de la Commission Internationale de l'Eclairage selon la recommandation 15:2004.

Le contraste ratio, ou couvrance, est calculé en faisant la moyenne arithmétique de Y sur fond noir, divisée par la valeur moyenne de Y sur fond blanc, multiplié par 100.

**Mesure de la couleur de la composition dans la masse**

La couleur dans la masse est mesurée après avoir rempli un contenant présentant une profondeur suffisamment importante pour pouvoir considérer une épaisseur de produit infinie au vu de la mesure, par exemple une profondeur de 3 mm ou plus.

Les coordonnées L*, a* et b* sont mesurées avec un spectrocolorimètre, par exemple de marque MINOLTA CM-2002 (D65/10°, mode composante spéculaire exclue).

**Mesure de la couleur après application**

La couleur est mesurée sur le fond sombre de la carte de contraste, la composition étant étalée comme pour la mesure de la couvrance ainsi que détaillé ci-dessus mais à une épaisseur de 150 μm au lieu de 50 μm pour les compositions liquides et les compositions solides non pulvérulentes, par exemple en stick.

L'écart ΔE est calculé comme suit :

$$\Delta E = \left[ \left( a^*_{dans\,la\,masse} - a^*_{après\,application} \right)^2 + \left( b^*_{dans\,la\,masse} - b^*_{après\,application} \right)^2 + \left( L^*_{dans\,la\,masse} - L^*_{après\,application} \right)^2 \right]^{1/2}$$

Lorsque l'écart ΔE varie en fonction de l'angle d'observation, en raison de la présence d'un agent de coloration goniochromatique, c'est l'écart maximal qui est retenu.

**Mesure de l'indice de blancheur**

L'indice de blancheur est calculé d'après les mesures de couleur à épaisseur infinie et selon la norme ASTM E313-05.

**Pigment interférentiel multicouche**

L'expression « pigment interférentiel multicouche » désigne un pigment capable de produire une couleur par un phénomène d'interférences entre les rayonnements lumineux réfléchis par une pluralité de couches superposées d'indices de réfraction différents, notamment une succession de couches de haut et de bas indices de réfraction. Le pigment peut comporter un substrat, par exemple de mica, recouvert d'une seule couche d'indice de réfraction différent, par exemple de Ti02.

Tous les pigments interférentiels multicouche peuvent être envisagés.

La couleur produite par le pigment interférentiel multicouche peut être quelconque, étant par exemple de longueur d'onde dominante comprise entre 580 et 650 nm ou non.

La composition peut comporter un seul pigment interférentiel multicouche ou plusieurs pigments interférentiels multicouche ayant des longueurs d'onde dominantes différentes.

Le pigment interférentiel muliticouche peut comporter un substrat (encore appelé coeur ou noyau) recouvert sur au moins une face d'une ou plusieurs couches en des matériaux et épaisseurs choisis de telle sorte qu'une couleur soit produite par interférences.

Des couches du pigment interférentiel peuvent entourer ou non le substrat, lequel peut présenter une forme aplatie ou non.

Le substrat peut comporter du mica naturel, du mica synthétique, du verre, de l'alumine, de la silice, ou encore un quelconque métal, alliage ou oxyde métallique. La nature du substrat pourra être choisie en fonction de la brillance souhaitée. Par exemple, pour un rendu très brillant, un substrat en verre ou en métal pourra être préféré.

Le pigment interférentiel peut comporter plus de quatre couches d'indices de réfraction différents.

La taille des particules du pigment interférentiel multicouche, donnée par la granulométrie moyenne à la moitié de la population, encore appelée $D_{50}$, va par exemple de 1 μm à 2000 μm, mieux de 5 μm à 2000 μm.

La proportion de pigment interférentiel multicouche est par exemple supérieure à 7 % et va par exemple de 7 à 20 % pour une composition non pulvérulente, liquide ou coulée, par exemple en stick et de 40 à 95 % pour une composition pulvérulente, libre ou compactée.

La couvrance de la composition peut être due essentiellement à la teneur en pigment interférentiel multicouche. En variante, au moins un pigment diffusant et/ou des charges peuvent conférer de la couvrance.

Les nacres sont des exemples de pigments interférentiels multicouche pouvant convenir.

<u>Nacres</u>

Par « nacre », il faut comprendre des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment ainsi que les pigments nacrés à base d'oxychlorure de bismuth.

Il peut également s'agir de particules de mica à la surface desquelles sont superposées au moins deux couches successives d'oxydes métalliques et/ou de matières colorantes organiques.

Les nacres peuvent présenter une couleur ou un reflet jaune, rose, rouge, bronze, orangé, brun, or et/ou cuivré.

A titre illustratif de nacres pouvant être introduites en tant que pigment interférentiel multicouche, on peut citer les nacres de couleur or notamment commercialisées par la société ENGELHARD sous le nom de Brillant gold 20 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne); les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Des pigments interférentiels multicouche présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO 25 TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

Le pigment interférentiel peut avantageusement être choisi parmi ceux conférant une couleur blanche dans la masse à la composition, à savoir les nacres notamment commercialisées par la société ENGELHARD sous le nom de SPARKLE 110P (Timica), Flamenco blue (Flamenco), Flamenco green (Flamenco), Flamenco red (Flamenco), Flamenco violet (Flamenco), Flamenco orange (Flamenco), Silkbalnc 110W (Timica), Extra large sparkle (Timica), Flamenco sparkle Gold (Flamenco), Flamenco sparkle green (Flamenco), Flamenco sparkle orange (Flamenco), Flamenco sparkle blue (Flamenco), Flamenco sparkle violet (Flamenco), Flamenco sparkle red (Flamenco), Flamenco summit gold (Flamenco) ; les nacres commercialisées par la société MERCK sous la dénomination Silk blue Timiron (Mica/TiO$_2$/SnO$_2$), Silk green Timiron (Mica/TiO$_2$/SnO$_2$), Silk red Timiron (Mica/TiO$_2$/SnO$_2$), Super red Timiron (Mica/TiO$_2$), Super green Timiron (Mica/TiO$_2$), Super blue Timiron (Mica/TiO$_2$), Artic Silver Timiron, Splendid copper Timiron (Mica/TiO$_2$/SiO$_2$), Splendid Violet Timiron (Mica/TiO$_2$/SiO$_2$) ; les nacres notamment commercialisées par la société ECKART sous la dénomination Prestige Silver (Prestige), Prestige Silver Star (Prestige), Prestige Gold (Prestige), Prestige soft gold (Prestige), Prestige silk green (Prestige), Prestige silk lilac (Prestige), Prestige silk blue (Prestige), Prestige silk red (Prestige).

Le pigment interférentiel peut être dépourvu de couche d'un matériau coloré, notamment choisi parmi FeOOH, Fe$_2$O$_3$, Cr$_2$O$_3$, TiO$_{2-x}$, TiO$_x$N$_y$, CrPO$_4$, KFe[Fe(CN)$_6$], Fe$_3$O$_4$, TiO, TiN, FeTiO$_3$, C, Ag, Au, Fe, Mo, Cr, W.

Le pigment interférentiel peut par exemple ne comporter sur le substrat qu'une ou plusieurs couches des

matériaux choisis parmi $TiO_2$ (rutile ou anatase), $ZrO_2$, $SnO_2$, $SiO_2$.

Le pigment interférentiel multicouche peut encore être choisi parmi les particules réfléchissantes interférentielles.

Particules réfléchissantes interférentielles

Ces particules peuvent être choisies parmi les particules à substrat synthétique enrobé au moins partiellement d'au moins une couche d'au moins un oxyde métallique, choisi par exemple parmi les oxydes de titane, notamment $TiO_2$, de fer notamment $Fe_2O_3$, d'étain, de chrome, le sulfate de baryum et les matériaux suivants : $MgF_2$, $CrF_3$, ZnS, ZnSe, $SiO_2$, $Al_2O_3$, MgO, $Y_2O_3$, $SeO_3$, SiO, $HfO_2$, $ZrO_2$, $CeO_2$, $Nb_2O_5$, $Ta_2O_5$, $MoS_2$ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Le pigment interférentiel multicouche peut encore être un pigment goniochromatique.

Pigment goniochromatique

Par « pigment goniochromatique », on désigne au sens de la présente invention un pigment permettant d'obtenir, lorsque la composition est étalée sur un support, un trajet de couleur dans le plan a\*b\* de l'espace colorimétrique CIE 1976 qui correspond à une variation Dh° de l'angle de teinte h° d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition a été étalée à l'état fluide avec une épaisseur de 300 $\mu$m au moyen d'un étaleur automatique sur une carte de contraste de marque ERICHSEN et de référence Typ 24/5, la mesure étant effectuée sur le fond noir de la carte.

Le pigment goniochromatique peut être choisi par exemple parmi les structures multicouches interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

La structure multicouche peut présenter ou non, par rapport à une couche centrale, une symétrie au niveau de la nature chimique des couches empilées.

Selon l'épaisseur et la nature des différentes couches, on obtient différents effets.

Des exemples de structures multicouche interférentielles symétriques sont par exemple les structures suivantes : $Fe_2O_3$/$SiO_2$/$Fe_2O_3$/$SiO_2$/$Fe_2O_3$, un pigment ayant cette structure étant commercialisé sous la dénomination SICOPEARL par la société BASF ; $MoS_2$/$SiO_2$/mica-oxyde/$SiO_2$/$MoS_2$ ; $Fe_2O_3$/$SiO_2$/mica-oxyde/$SiO_2$/$Fe_2O_3$ ; $TiO_2$/$SiO_2$/$TiO_2$ et $TiO_2$/$Al_2O_3$/$TiO_2$, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE® HC par la société WACKER.

Comme pigment goniochromatique, on peut encore utiliser certaines nacres, des pigments à effets sur substrat synthétique, notamment substrat type alumine, silice, borosilicate, oxyde de fer, aluminium, ou des paillettes interférentielles issues d'un film de polytéréphtalate.

Le matériau peut en outre comporter des fibres goniochromatiques dispersées. De telles fibres pourront présenter une longueur inférieure à 80 $\mu$m par exemple.

**Milieu cosmétiquement acceptable**

Par « milieu cosmétiquement acceptable », on désigne un milieu non toxique susceptible d'être appliqué sur les matières kératiniques d'êtres humains.

Le milieu cosmétiquement acceptable sera adapté à la nature du support sur lequel doit être appliqué la composition ainsi qu'à la forme sous laquelle la composition est destinée à être conditionnée.

La composition selon l'invention peut comprendre un milieu aqueux et/ou une phase grasse, être anhydre ou non.

Phase aqueuse ou grasse

La composition peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols.

La phase hydrophile peut, en outre, contenir des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0 % à 90 %, notamment 0,1 % à 90 % en poids, par rapport au poids total de la composition, et de préférence de 0 % à 60 % en poids, notamment 0,1 % à 60 % en poids.

La composition peut également comprendre une phase grasse, notamment constituée de corps gras liquides à 25 °C et éventuellement de corps gras solides à température ambiante tels que les cires, les corps gras pâteux, les gommes et leurs mélanges.

Comme corps gras liquides à température ambiante, appelés souvent huiles, utilisables dans l'invention, on peut citer entre autres les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et, notamment les isoparaffines en $C_8$-$C_{16}$ telles que l'isododé-cane, l'isodécane, l'isohexadécane, la vaseline, les polydécènes, le polyisobutène hydrogéné tel que le Parléam®, le squalane; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 % par rapport au poids total de la composition.

La composition selon l'invention peut également comprendre un ou plusieurs solvants organiques, physiologiquement acceptables. Ce ou ces solvants, qui peuvent être lipophiles, peuvent être présents en une teneur allant de 0 à 90 %, mieux de 0 à 60% en poids, par rapport au poids total de la composition et encore mieux de 0,1 à 30%.

Le milieu peut comporter une phase organique liquide dans laquelle de l'eau est dispersée ou émulsionnée.

La composition peut encore présenter une phase grasse continue, pouvant contenir moins de 5% d'eau, notamment moins de 1% d'eau par rapport à son poids total et en particulier être sous forme anhydre.


Agent filmogène

Le milieu peut comporter un agent filmogène, notamment un polymère filmogène.

On entend par « agent filmogène » un agent apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconée.

La composition peut comporter une phase aqueuse et le polymère filmogène peut être présent dans cette phase aqueuse. Celui-ci pourra être un polymère en dispersion ou en solution.

La composition peut comporter une phase huileuse et le polymère filmogène peut être présent dans cette phase huileuse. Le polymère pourra alors être en dispersion ou en solution.

Parmi les polymères filmogènes utilisables, on peut citer les polymères synthétiques, de type radicalaire ou de type polycondensat, les polymères d'origine naturelle, et leurs mélanges.

Les polymères filmogènes de type radicalaire peuvent être notamment des polymères, ou des copolymères, vinyliques, notamment des polymères acryliques.

Parmi les polycondensats filmogènes, on peut citer les polyuréthanes, les polyesters, les polyesters amides, les polyamides, et les résines époxyesters, les polyurées.

Les polyesters peuvent être obtenus, de façon connue, par polycondensation d'acides dicarboxyliques avec des polyols, notamment des diols.

Les polyesters amides peuvent être obtenus de manière analogue aux polyesters, par polycondensation de diacides avec des diamines ou des amino alcools.

A titre d'exemple de polymères filmogènes liposolubles, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyl éther, stéarate de vinyle/acétate d'allyle, diméthyl-2, 2 octanoate de vinyle/laurate de vinyle, diméthyl-2, 2 pentanoate d'allyle/ laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Le polymère filmogène peut encore être choisi parmi les résines de silicone, généralement solubles ou gonflables dans les huiles de silicone, qui sont des polymères de polyorganosiloxanes réticulés.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Bien entendu, cette liste de polymères n'est pas exhaustive.

Autres agents de coloration

La composition peut comporter un ou plusieurs pigments diffusants, générant une couleur par un phénomène d'absorption, dans une proportion permettant de conserver le phénomène d'interférences responsable de la couleur de la composition une fois appliquée sur les matières kératiniques.

La composition peut ne pas comporter d'autres agents de coloration que le ou les pigment(s) interférentiel(s) multicouche, notamment être dépourvue de pigments à base d'oxydes de fer ou autres pigments générant une couleur par un phénomène d'absorption.

La composition peut contenir moins de 0,5% en masse de pigments générant une couleur par absorption, notamment moins de 0,5% de pigments à base d'oxydes de fer, mieux moins de 0,2%.

Lorsque des pigments diffusants sont présents, divers pigments diffusants peuvent être envisagés, étant par exemple choisi parmi les laques ou pigments organiques sélectionnés notamment parmi les matériaux ci-dessous et leurs mélanges:

- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La laque peut être supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

Le pigment diffusant peut être un pigment composite, comportant un noyau enrobé au moins partiellement par une écorce. Un tel pigment composite peut être composé notamment de particules comportant un noyau inorganique et au moins un enrobage au moins partiel d'au moins une matière colorante organique. Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5. Un pigment composite peut présenter par exemple une surface spécifique comprise entre 1 et 1000 $m^2$/g, notamment entre 10 et 600 $m^2$/g environ, et en particulier entre 20 et 400 $m^2$/g environ. La surface spécifique est la valeur mesurée par la méthode BET. La proportion massique du noyau peut excéder 50 % par rapport au poids total du pigment composite, par exemple aller de 50 % à 70 %, par exemple de 60 % à 70 %.

L'agent de coloration peut encore être un colorant.

Il peut s'agir d'un colorant d'origine végétale, animale ou minérale, en particulier d'origine végétale ou minérale, notamment d'origine végétale. Ce colorant peut être de nature non synthétique.

Le colorant peut être un colorant naturel hydrosoluble ou liposoluble.

A titre illustratif des agents de coloration naturels hydrosolubles susceptibles d'être mis en oeuvre selon l'invention, on peut particulièrement citer le caramel, le jus de betterave et le carmin, la bétanine (betterave), la chlorophylline cuivrée, le bleu de méthylène, les anthocyanines (enocianine, carotte noire, hibiscus, sureau) et la riboflavine.

A titre illustratif des agents de coloration naturels liposolubles susceptibles d'être mis en oeuvre selon l'invention, on peut particulièrement citer le rouge Soudan, le β-carotène, les caroténoïdes, le lycopène, l'huile de palme, le brun Soudan, le jaune quinoléique les xanthophylles (capsanthine, capsorubine, lutéine), et le curcumin.

Comme autres colorants naturels, on peut plus particulièrement citer les anthocyanes de fleurs ou de fruits ou leurs dérivés, les flavonoïdes et tanins extraits de végétaux natifs ou fermentés, la juglone, la lawsone, les extraits de soja fermenté, d'algues, de champignons, de micro-organismes, les sels de Flavylium non substitués en position 3 tels que décrit dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Comme exemple de colorants synthétiques, on peut citer les colorants liposolubles synthétiques tels que, par exemple, le DC Red 17, le DC Red 21, le DC Red 27, le DC Green 6, le DC Yellow 11, le DC Violet 2 et le DC Orange 5.

Comme exemple de colorants hydrosolubles synthétiques, on peut citer le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5 et le FDC Blue 1.

Charges

La composition cosmétique peut comprendre des charges.

Par « charges », on désigne des particules de toute forme insolubles dans le milieu de la composition, quelle que soit la température à laquelle la composition est fabriquée. Ces charges peuvent servir notamment à modifier la rhéologie ou la texture de la composition.

A titre d'exemple de charges, on peut citer, entre autres, le talc, le mica, la silice, le kaolin, et les poudres de polyamide (Nylon® ou Orgasol® de chez Atochem).

La teneur en charges sera choisie de façon à ne pas entraver outre mesure le résultat recherché.

Actifs et autres composés

La composition cosmétique peut également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, antirides...). Ces actifs peuvent être utilisés par exemple à des concentrations de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

**Formes galéniques**

La composition cosmétique peut se présenter sous toute forme galénique normalement utilisée pour une application topique et notamment sous forme anhydre ou non anhydre, sous forme solide, pulvérulente libre ou compactée, coulée, par exemple en stick, liquide, d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules situés à l'interface huile/eau ou d'un spray.

Par « composition anhydre », on désigne une composition ayant moins de 5 % en masse d'eau, mieux moins de 3 %, voire moins de 1 %. Une composition anhydre peut ne pas comporter d'eau ajoutée intentionnellement au cours de la préparation de la composition.

La composition peut notamment se présenter sous forme de stick.

La composition cosmétique peut constituer, entre autres produits de maquillage, un rouge à lèvres en stick ou liquide, un brillant à lèvres liquide, une pâte de rouge à lèvres, un fard à joues, un fond de teint, un produit de contour des yeux, un eye-liner, un mascara, un vernis à ongles, une ombre à paupières, une base de maquillage, et plus généralement tout produit de maquillage du corps ou des cheveux.

La composition de l'invention peut être obtenue selon les procédés de préparation classiquement utilisés en cosmétique.

**Conditionnement et modes d'application**

La composition peut être conditionnée dans tout réceptacle ou sur tout support prévu à cet effet.

La composition peut être appliquée en utilisant un applicateur, floqué ou non, par exemple une mousse, un embout, un pinceau, un feutre, une spatule, un fritté, une brosse, un peigne, un tissé ou non tissé.

L'application peut encore s'effectuer avec le doigt ou en déposant directement la composition sur le support à maquiller, par exemple par friction dans le cas d'un stick ou par pulvérisation ou projection à l'aide d'un dispositif piézoélectrique ou par transfert d'une couche de composition préalablement déposée sur un support intermédiaire.

La composition peut, le cas échéant, être appliquée comme couche de base (base coat) recouverte d'une couche d'une autre composition (top coat), destinée par exemple à conférer de la brillance, ou comme couche de revêtement sur une couche d'une autre composition, voire entre une couche de base et une couche de revêtement.

La composition peut être conditionnée dans un dispositif de conditionnement qui laisse apparaître la couleur de la composition dans la masse.

Il peut s'agir d'un récipient ayant un corps au moins partiellement transparent et/ou d'un dispositif de conditionnement comportant un organe de bouchage au moins partiellement transparent.

La composition peut être conditionnée dans un dispositif qui permet de voir à la fois la couleur de la composition dans la masse et la couleur de la composition après application sur des matières kératiniques.

La composition peut encore être conditionnée dans un dispositif qui comporte des moyens représentatifs de la couleur révélée à l'application, par exemple un dépôt d'une couche de la composition ou d'une encre ou vernis comportant les mêmes agents de coloration que la composition.

L'invention a encore pour objet une composition ainsi conditionnée.

Le cas échéant, la composition peut être conditionnée avec un aimant permettant de modifier l'orientation des particules de pigment interférentiel multicouche et/ou les particules réfléchissantes du pigment à réflexion métallique, lorsque l'un de ces pigments au moins présente une susceptibilité magnétique non nulle.

**Procédé de présentation et présentoir**

L'invention a encore pour objet, selon un autre de ses aspects, un procédé de présentation d'une composition cosmétique réalisée conformément à l'invention, comportant les étapes consistant à :

- éclairer au moins une première région d'un échantillon de composition avec un éclairage diffus de façon à faire apparaître la couleur dans la masse,
- éclairer au moins une deuxième région d'un échantillon de composition avec un éclairage directionnel de façon à faire apparaître la couleur interférentielle.

Les première et deuxième régions peuvent être des régions différentes d'un même échantillon de composition.

En variante, les première et deuxième régions appartiennent à des échantillons différents.

En variante encore, les première et deuxième régions correspondent à une même région et les éclairages diffus et directionnel sont appliqués alternativement.

L'invention a encore pour objet, selon un autre de ses aspects, un présentoir comportant :

- une source d'éclairage directionnel,
- une source d'éclairage diffus,
- un support pour permettre d'exposer au moins un échantillon de composition à la source d'éclairage diffus et au moins un échantillon de composition à la source d'éclairage directionnel.

**Procédé de maquillage**

L'invention a encore pour objet un procédé de maquillage des matières kératiniques au moyen d'une composition selon l'invention.

Il peut s'agir du maquillage de la peau, des lèvres, des ongles, des cils, sourcils ou cheveux.

**Kit**

La présente invention a également pour objet un kit de maquillage comportant:

- une première composition selon l'invention,
- une deuxième composition comportant un milieu cosmétiquement acceptable et à appliquer sous ou sur la première composition.

Cette deuxième composition est par exemple destinée à améliorer la tenue de la première composition et/ou à en modifier l'aspect.

**Exemples proposés**

Les proportions indiquées sont massiques.

Exemple 1 : Brillant à lèvres

| Octyl-2 dodécanol | 0 |
|---|---|
| Ditertiobutyl 4-hydroxytoluène | ,07 |
| Polybutène (monooléfines / isoparaffines 95/5) (PM : 2060) | 7 |
| Mélange de p-hydroxybenzoates d'isopropyle, iso-butyle, n-butyle (40/30/30) | ,6 |
| Tétra-iso-stéarate de pentaérythrityle | 1,33 |
| Tri-méllitate de tridécyle | 1 |
| Triglycéride d'acide 2-décyl tétradécanoïque (GUERBET C24) | 5 |
| Pigment interférentiel multicouche * | 5 |
| * TIMIRON SILK RED de la société MERCK | |

L'effet produit par ce brillant à lèvres résulte de l'association de plusieurs phénomènes. Le premier concerne le rendu coloriel à l'application qui est intense et couvrant. A cela s'ajoute la brillance du film qui est le fruit de la présence du système huile/polymère. Ce brillant à lèvres présente une couleur de masse qui est blanche.

Exemple 2 : Rouge à lèvres

| Tri-méllitate de tri-décyle | 0,8 |
|---|---|
| Lanoline liquide | 0 |
| Malate de d'iso-stéaryle | 1 |
| Lanoline acétylène | 0 |
| Triglycérides d'acides laurique/palmitique/cérylique/stéarique (50/20/10/10) | |
| Cire microcristalline (C20-C60) | |
| Lanolate d'isopropyle protégé | 0 |
| Octyl-2-decanol | 3,5 |
| Phényl trimethylsiloxy trisiloxane (VISCOSITE: 20 CST - PM: 372) | |
| Cire de polyethylène (PM :500) | |
| Pigment interférentiel multicouche * | 4,7 |
| * TIMIRON SILK RED de la société MERCK | |

L'effet produit par ce rouge à lèvres résulte de l'association de plusieurs phénomènes. Le premier concerne le rendu coloriel à l'application qui est intense et couvrant. A cela s'ajoute la texture du film qui est le fruit de la présence du système huile/polymère/cire. Ce rouge à lèvres présente une couleur de masse qui est blanche.
Ce rouge à lèvres produit un résultat maquillage, notamment en terme de couvrance, tout à fait satisfaisant et il n'est pas nécessaire d'appliquer une autre couche de maquillage pour révéler son effet.

La figure ci-dessus permet la comparaison des couvrances obtenues

a) à gauche avec la composition cosmétique de l'exemple 2

b) à droite avec une formulation similaire mais ne contenant que 3% du pigment interférentiel multicouche.

Exemple 3 : Blush

| | |
|---|---|
| Triéthano lamine | |
| Acide éthylène diamine tétracétique, sel disodique, 2H2O | ,2 |
| Homopolymère carboxyvinylique réticulée | ,5 |
| Polyvinylpyrro lidone | ,6 |
| Glycérol | ,75 |
| Eau désionisée | 0,45 |
| 1,3 butylène glycol | |
| Microsphère de silice (3μm) | ,5 |
| Pigment interférentiel multicouche * | |
| * TIMIRON SILK BLUE de la société MERCK | |

L'effet produit par ce blush résulte de l'association de plusieurs phénomènes. Le premier concerne le rendu coloriel à l'application qui est intense et couvrant. A cela s'ajoute la texture du film qui est le fruit de la présence du système aqueux.
Ce blush présente une couleur de masse qui est blanche.
Le résultat maquillage, notamment en terme de couvrance, est tout à fait satisfaisant et il n'est pas nécessaire d'appliquer une autre couche de maquillage pour révéler son effet. Ce blush permet de souligner d'un bleu très intense les pommettes.

Exemple 4 : Vernis à ongles à base aqueuse

| Pyophosphate tétrasodique | ,2 |
|---|---|
| Polydimethylsiloxane oxyethylène a terminaisons méthoxyl | ,5 |
| Mélange de polyuréthane aliphatique, N-méthyl pyrrolidone, triéthylamine, eau (35/8,5/2/54,5) | 8 |
| Glycérol | |
| Eau désionisée | 5 |
| Alcool éthylique (96°) | ,8 |
| Laponite synthétique (silicate mixte magnésium/lithium/sodium) | ,3 |
| Pigment interférentiel multicouche * | 1,2 |
| * TIMIRON SILK GREEN de la société MERCK | |

L'effet produit par ce vernis à ongles résulte de l'association de plusieurs phénomènes. Le premier concerne le rendu coloriel à l'application qui est intense et couvrant. A cela s'ajoute la texture du film qui est le fruit de la présence du système aqueux. Ce vernis à ongles présente une couleur de masse qui est blanche. Le résultat maquillage, notamment en terme de couvrance, est tout à fait satisfaisant et il n'est pas nécessaire d'appliquer une autre couche de maquillage pour révéler son effet.

Exemple 5 : Vernis à ongles anhydre

| Nitrocellulose | 1 |
|---|---|
| N-éthyl o,p-toluènesulfonamide | |
| Résine alkyde | 0 |
| Isopropanol | |
| Pigment interférentiel multicouche * | 0 |
| Acétate de butyle/acétate d'éthyle 50/50 | Qsp 100 |
| * TIMIRON SILK RED de la société MERCK | |

L'effet produit par ce vernis à ongles résulte de l'association de plusieurs phénomènes. Le premier concerne le rendu coloriel à l'application qui est intense et couvrant. A cela s'ajoute la texture du film qui est le fruit de la présence du système solvant.

Ce vernis à ongles présente une couleur de masse qui est blanche. Le résultat maquillage, notamment en terme de couvrance, est tout à fait satisfaisant et il n'est pas nécessaire d'appliquer une autre couche de maquillage pour révéler son effet.

Exemple 6 : Fard à paupière pulvérulent

| TERTIOBUTYL 4-HYDROXYANISOLE | 0,012 |
|---|---|
| DITERTIOBUTYL 4-HYDROXYTOLUENE | 0,012 |
| VASELINE BLANCHE | 1,2 |
| ALCOOL OLEIQUE | 1,2 |

(suite)

| | |
|---|---|
| LANOLINE LIQUIDE PROTEGEE | 0,66 |
| HUILE DE VASELINE | 6,516 |
| HUILE DE RICIN | 1,296 |
| MYRISTATE D'ISOPROPYLE | 0,864 |
| P-HYDROXYBENZOATE DE PROPYLE | 0,24 |
| MELANGE P-HYDROXYBENZOATES DE METHYLE, ETHYLE, PROPYLE, BUTYLE ISOBUTYLE / PHENOXY-2 ETHANOL | 0,6 |
| pigment multicouche intérférentiel* | 53,3 |
| STEARATE DE MAGNESIUM | 4 |
| TALC | 30,1 |
| * TIMIRON SILK RED de la société MERCK | |

L'effet produit par ce fard à paupières poudre résulte de l'association de plusieurs phénomènes. Le premier concerne le rendu coloriel à l'application qui est intense et couvrant. A cela s'ajoute la texture du dépôt qui est le fruit de la présence du système pigment interférentiel multicouche/charges/liant. Ce fard à paupières présente une couleur de masse qui est blanche. Le résultat maquillage, notamment en terme de couvrance, est tout à fait satisfaisant et il n'est pas nécessaire d'appliquer une autre couche de maquillage pour révéler son effet.

Exemple 7 : Fards à paupière liquide

| | |
|---|---|
| DITERTIOBUTYL 4-HYDROXYTOLUENE | ,09 |
| HECTORITE MODIFIEE DISTEARYL DIMETHYL AMMONIUM | ,74 |
| TRIGLYCERIDES D'ACIDES LAURIQUE/PALMITIQUE/CETYLIQUE/STEARIQUE (50/20/10/10) | ,46 |
| CARBONATE DE PROPYLENE | ,22 |
| CIRE D'ABEILLE BLANCHIE | ,77 |
| BEURRE DE SHOREA PROTEGE | ,7 |
| FRACTION LIQUIDE DE BEURRE DE KARITE PROTEGEE | ,85 |
| POUDRE DE NYLON 12 | 0,4 |
| ISO-DODECANE | 8,32 |
| P-HYDROXYBENZOATE DE PROPYLE | ,17 |
| PARAFFINE RAFFINEE PROTEGEE | ,88 |
| TALC | 0,4 |
| Pigment interférentiel multicouche* | 5 |
| * TIMIRON SILK BLUE de la société MERCK | |

L'effet produit par ce fard à paupières résulte de l'association de plusieurs phénomènes. Le premier concerne le rendu coloriel à l'application qui est intense et couvrant. A cela s'ajoute la texture du film qui est le fruit de la présence du système solvant/polymère/cire/charge.

Le fard à paupières présente une couleur de masse qui est blanche. Le résultat maquillage, notamment en terme de couvrance, est tout à fait satisfaisant et il n'est pas nécessaire d'appliquer une autre couche de maquillage pour révéler son effet.

Ce fard permet de souligner d'un bleu très intense les paupières.

On peut associer la composition selon l'invention à une composition de recouvrement ou de base. On décrit ci-dessous des exemples d'associations.

Exemple 8 : Rouge à lèvres

Première composition

| Tri-mellitate de tri-décyle | 11 |
|---|---|
| Lanoline liquide | 10 |
| Malate de d'iso-stéaryle | 13 |
| Lanoline acétylène | 10 |
| Triglycérides d'acides laurique/palmitique/cétylique/stéarique (50/20/10/10) | 5 |
| Cire microcristalline (C20-C60) | 3 |
| Lanolate d'isopropyle protégé | 10 |
| Octyl-2-decanol | 16 |
| Phényl triméthylsiloxy trisiloxane (VISCOSITE: 20 CST - PM: 372) | 4 |
| Cire de polyéthylène (PM :500) | 8 |
| Pigment interférentiel multicouche* | 10 |
| * TIMIRON SILK RED de la société MERCK | |

Deuxième composition (de recouvrement)

| Octyl-2 dodécanol | 0 |
|---|---|
| Ditertiobutyl 4-hydroxytoluène | ,07 |
| Polybutène (monooléfines / isoparaffines 95 / 5) (PM : 2060) | 0 |
| Mélange de p-hydroxybenzoates d'isopropyle, iso-butyle, n-butyle (40/30/30) | ,4 |
| Tétra-iso-stéarate de pentaérythrityle | 1,33 |
| Tri-mellitate de tridécyle | 2 |
| Triglycéride d'acide 2-décyl tétradécanoïque (GUERBET C24) | 1 |
| Pigment à effet** | |
| ** METASHINE ME 2040 PS de la société NIPPON SHEET GLASS | |

La première composition permet de générer une couche de maquillage homogène d'une couleur rouge très intense. La seconde composition permet de créer un effet loupe agrémenté de point de surbrillance argent qui donnent du relief à l'ensemble du résultat maquillage.

Exemple 9 : Rouge à lèvres

Composition de base

| Tri-mellitate de tri-décyle | 12 |
|---|---|
| Lanoline liquide | 11 |
| Malate de d'iso-stéaryle | 14 |
| Lanoline acétylène | 11 |
| Triglycérides d'acides laurique/palmitique/cétylique/stéarique (50/20/10/10) | 6 |
| Cire microcristalline (C20-C60) | 3 |

(suite)

| Lanolate d'isopropyle protégé | 10 |
|---|---|
| Octyl-2-decanol | 16 |
| Phényl triméthylsiloxy trisiloxane (VISCOSITE: 20 CST - PM: 372) | 4 |
| Cire de polyéthylène (PM :500) | 8 |
| Pigment noir d'oxyde de Fer $Fe_3O_4$ | 5 |

Composition contenant le pigment interférentiel multicouche

| Octyl-2 dodécanol | 10 |
|---|---|
| Ditertiobutyl 4-hydroxytoluène | 0,07 |
| Polybutène (monooléfines / isoparaffines 95 / 5) (PM : 2060) | 45,2 |
| Mélange de p-hydroxybenzoates d'isopropyle, iso-butyle, n-butyle (40/30/30) | 0,4 |
| Tétra-iso-stéarate de pentaérythrityle | 11,33 |
| Tri-mellitate de tridécyle | 12 |
| Triglycéride d'acide 2-décyl tétradécanoïque (GUERBET C24) | 11 |
| Pigment interférentiel multicouche** | 10 |
| ** TIMIRON SILK RED de la société MERCK | |

La composition de base est appliquée en premier et permet de générer une couche de maquillage homogène d'une couleur noire très intense. La composition contenant le pigment interférentiel multicouche est appliquée ensuite et permet de créer un effet coloré donnant un résultat maquillage très intense.

La description qui suit se rapporte à un autre objet de l'invention.

**Stick multicolore**

Mesure de la couvrance du produit solide

Les compositions du produit solide sont fondues et mélangées, puis la couvrance est mesurée comme indiqué plus haut pour une composition unique.

Composé pâteux

Le milieu cosmétiquement acceptable du produit solide de l'un ou l'autre des blocs peut comporter un composé pâteux choisi par exemple parmi :

- les composés siliconés polymères ou non,
- les composés fluorés polymères ou non,
- les polymères vinyliques, notamment:

    - les homopolymères d'oléfines
    - les copolymères d'oléfines
    - les homopolymères et copolymères de diènes hydrogénés
    - les oligomères linéaires ou ramifiés, homo ou copolymères de (méth)acrylates d'alkyle ayant de préférence un groupement alkyle en $C_8$-$C_{30}$
    - les oligomères homo et copolymères d'esters vinyliques ayant des groupements alkyles en $C_8$-$C_{30}$
    - les oligomères homo et copolymères de vinyléthers ayant des groupements alkyles en $C_8$-$C_{30}$,

- les polyéthers liposolubles résultant de la polyéthérification entre un ou plusieurs diols en $C_2$-$C_{100}$, de préférence en $C_2$-$C_{50}$,

- les esters,
- et leurs mélanges.

Le composé pâteux est de préférence un polymère, notamment hydrocarboné.

Composés pâteux siliconés et /ou fluorés

Un exemple de composé pâteux siliconé et fluoré est le polyméthyl trifluoropropryl méthylalkyl diméthylsiloxane, fabriqué sous la dénomination X22-1088 par la société SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Composés pâteux polyéthers

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéariques, à l'image notamment de ceux commercialisés sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique; et leurs mélanges.

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.

L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :

a) les esters partiels ou totaux de monoacides carboxyliques aliphatiques mono hydroxylés linéaires, saturés;
b) les esters partiels ou totaux de monoacides carboxyliques aliphatiques mono hydroxylés insaturés ;
c) les esters partiels ou totaux de polyacides carboxyliques aliphatiques mono hydroxylés saturés;
d) les esters partiels ou totaux de polyacides carboxyliques aliphatiques poly hydroxylés saturés ;
e) les esters partiels ou totaux de polyols aliphatiques en $C_2$ à $C_{16}$ ayant réagi avec un mono ou un poly acide carboxylique aliphatique mono ou poly hydroxylé,

et leurs mélanges.

Les esters aliphatiques d'ester sont avantageusement choisis parmi:

- l'ester résultant de la réaction d'estérification de l'huile de ricin hydrogénée avec l'acide isostéarique dans les proportions 1 pour 1 (1/1) ou monoisostéarate d'huile de ricin hydrogénée,

- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

Le composé pâteux représente de préférence 1 à 99%, mieux 1 à 60%, mieux 2 à 30% et mieux encore 5 à 15% en poids de chaque composition du produit solide.

En particulier, un composé pâteux et un ester tels que définis ci-dessus peuvent être associés dans la composition cosmétique dans un rapport pondéral composé pâteux/ester variant de 0,25 à 0,75 notamment de 0, 3 à 0,6.

La composition cosmétique selon l'invention peut présenter une phase grasse continue, pouvant contenir moins de 5 % d'eau, notamment moins de 1 % d'eau par rapport à son poids total.

Phase grasse

Chaque composition, notamment lorsque le produit est destiné à être appliqué sur les lèvres, peut comporter au moins une phase grasse et notamment au moins un corps gras liquide à température ambiante (25 °C) et à pression atmosphérique et/ou un corps gras solide à température ambiante et à pression atmosphérique tel que les cires, les gommes et leurs mélanges.

La phase grasse peut en outre contenir des agents gélifiants et structurants d'huiles de nature organique et/ou des solvants organiques lipophiles.

La phase grasse de la composition selon l'invention peut comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en C8-C16 tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10-6 m2/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 % à 98 % en poids, notamment de 1 % à 65 % en poids, et en particulier de 2 % à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
les huiles hydrocarbonées d'origine animale,

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/ caprique comme ceux vendus par la société Stéarineries Dubois ou ceux vendus sous les dénominations Miglyol 1810®, 812® et 818® par la société Dynamit Nobel,
- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq$ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans une composition d'un produit selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates et leurs mélanges.

Les huiles non volatiles peuvent être présentes dans une composition d'un produit selon l'invention en une teneur allant de 0,01 à 90 % en poids, notamment de 0,1 % à 85% en poids, et en particulier de 1 % à 70 % en poids, par rapport au poids total de la composition.

Les huiles peuvent représenter de 0 à 99 % du poids total de chaque composition, de préférence de 0,05 à 60 % et mieux de 1 à 35 %.

Les huiles peuvent être de poids moléculaire compris entre 650 et 10.000 g/mol, de préférence entre 750 et 7.500 g/mol.

Selon un mode de mise en oeuvre, chaque composition comprend une phase huileuse comprenant au moins 70 % en poids d'une huile de masse molaire comprise entre 650 et 10.000 g/mol, de préférence entre 750 et 7.500 g/mol. La phase huileuse comprend avantageusement plus de 80 %, de préférence plus de 85 % en poids d'un huile de masse molaire comprise entre 650 et 10.000 g/mol, de préférence entre 750 et 7.500 g/mol.

L'huile de masse molaire élevée peut être choisie parmi les polymères lipophiles :

- les esters d'acides gras linéaires ayant un nombre total de carbones allant de 35 à 70,
- les esters hydroxylés,
- les esters aromatiques,
- les esters d'alcool gras ou d'acides gras ramifiés en C24-C28,
- les huiles siliconées,
- les huiles d'origine végétale,
- et leurs mélanges.

L'huile de masse molaire élevée peut être choisie parmi les polybutylènes, les polyisobutylènes hydrogénés, les polydécènes, les polydécènes hydrogénés, les copolymères de la vinylpyrrolidone tel que le PVP / héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tétraisostéarate de pentaérythrityle, le tri décyl-2 tétradécanoate de glycéryle, le tétra décyl-2 tétradécanoate de pentaérythrityle, les silicones phénylées, l'huile de sésame, et leurs mélanges.

Chaque composition peut également comporter un corps gras solide à température ambiante et à pression atmosphérique, choisi par exemple parmi les cires, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Cires et gommes

Chaque composition peut contenir au moins une cire.

Par "cire", on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

Les gommes utilisables dans l'invention se présentent généralement sous forme solubilisée dans une huile, les polymères sont solides à température ambiante et les résines peuvent être liquides ou solides à température ambiante.

Par « gomme » on entend un corps gras qui se présente sous forme de polymère solide à température ambiante ayant un poids moléculaire moyen en poids de 50 000 à 1 000 000. La gomme est souvent vendue en dispersion dans un solvant organique, du type huile de silicone.

La nature et la quantité des gommes ou cires sont fonction des propriétés mécaniques et des textures recherchées. A titre indicatif, la cire peut représenter de 0,01 à 50 %, de préférence de 2 à 40 %, et mieux de 5 à 30 % du poids total de chaque composition du produit.

En particulier, la cire peut être présente sous forme d'émulsion cire(s)-dans-eau.

La cire peut être présente dans chaque composition en une teneur allant de 0,01 % à 50 % en poids, en particulier de 0,1 % à 30 % en poids, et notamment de 0,2 % à 20 % en poids, par rapport au poids total de la composition.

Préparation

Chaque composition du produit solide de l'un ou l'autre des blocs peut être obtenue selon les procédés de préparation classiquement utilisés en cosmétique ou en dermatologie.

Le produit peut se présenter sous une forme extrudée, compactée ou coulée en stick ou en coupelle. Les compositions du produit peuvent être coextrudées.

Le produit peut constituer, entre autres produits de maquillage, un produit à appliquer sur les lèvres, par exemple un brillant à lèvres, un rouge à lèvres, un baume à lèvres, un fard à joues, un fond de teint, un produit de contour des yeux, un eye-liner, une ombre à paupières, une base de maquillage, un produit de maquillage du corps ou des cheveux.

Conditionnement et modes d'application

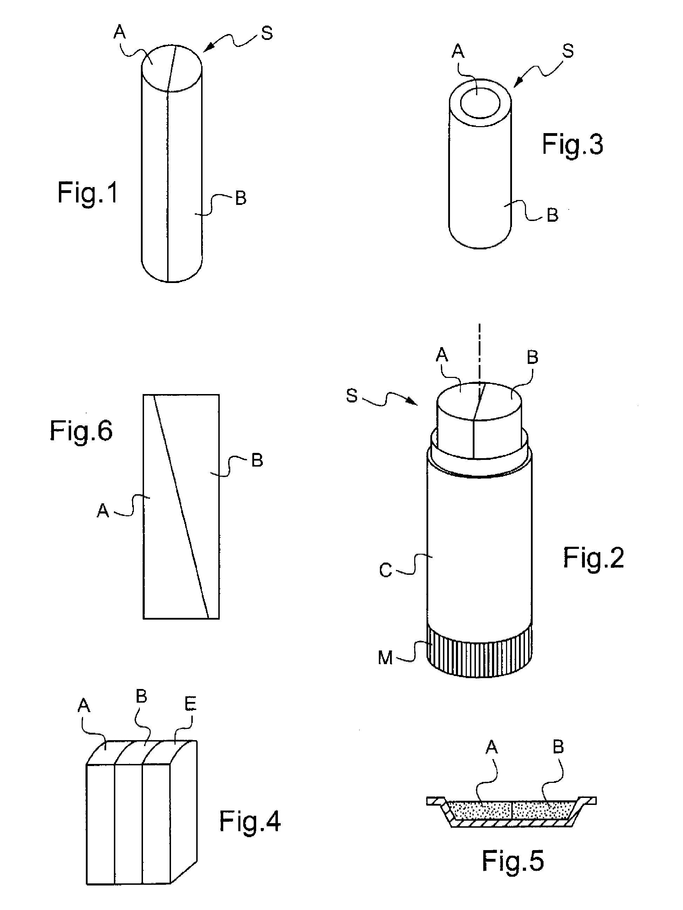

On a représenté à titre d'exemple à la figure 1 un stick S qui comporte deux blocs A et B s'étendant sur toute la longueur du stick.

Chaque bloc A ou B occupe par exemple la moitié du stick et ces deux blocs sont par exemple accolés selon le diamètre du stick.

Le stick S peut être conditionné dans un dispositif de conditionnement et de distribution comportant par exemple un corps C dans lequel le stick est reçu au moins partiellement, comme illustré à la figure 2, et un mécanisme d'entraînement M permettant de déplacer le stick relativement au corps C.

Le dispositif de conditionnement et de distribution peut être de tout type adapté au conditionnement d'un stick et le mécanisme d'entraînement M comporte par exemple une molette rotative en partie inférieure.

Les deux blocs A et B peuvent être de même proportion ou non au sein du stick.

L'un des blocs peut être entouré par l'autre bloc, comme illustré à la figure 3.

Les deux blocs A et B peuvent chacun présenter un écart de couleur ΔE entre la couleur au sein du stick et la couleur après application sur les matières kératiniques.

Seul l'un des blocs A ou B peut présenter cette caractéristique, l'autre bloc ne présentant pas par exemple cette propriété.

Les deux blocs A et B peuvent présenter ou non sensiblement la même couleur au sein du stick, par exemple la couleur blanche.

Le stick peut comporter, le cas échéant, plus de deux blocs, par exemple trois blocs comme illustré à la figure 4. Le troisième bloc E peut ou non présenter un écart de couleur entre la couleur au sein du stick, dans la masse, et la couleur après application sur les matières kératiniques.

Le stick peut être réalisé par co-extrusion, coulage ou compactage des compositions destinées à former les différents blocs.

Dans l'exemple de la figure 5, le produit comporte deux blocs A et B coulés ou compactés dans une coupelle.

Dans une variante non illustrée, les blocs de composition sont contenus dans une gaine de manière à former un crayon.

Chaque bloc A ou B peut avoir une section transversale constante sur toute la largeur du produit. En variante, l'un des blocs au moins peut présenter une section transversale variable le long de la longueur du produit.

L'un des blocs peut présenter une section transversale circulaire.

Un bloc peut présenter, en coupe longitudinale, une forme en biseau s'élargissant vers une extrémité longitudinale tandis que l'autre bloc présente également une forme en biseau, s'élargissant dans le sens inverse, comme illustré en coupe longitudinale à la figure 6.

Procédé de présentation et présentoir

L'invention a encore pour objet, selon un autre de ses aspects, indépendamment ou en combinaison avec ce qui précède, un procédé de présentation d'une composition cosmétique réalisée conformément à l'invention, comportant les étapes consistant à :

- éclairer au moins une première région d'un échantillon de composition avec un éclairage diffus de façon à faire apparaître la couleur dans la masse,
- éclairer au moins une deuxième région d'un échantillon de composition avec un éclairage directionnel de façon à faire apparaître la couleur interférentielle.

Le produit peut être éclairé de façon à faire apparaître simultanément toutes les couleurs des compositions après application.

Les première et deuxième régions peuvent être des régions différentes d'un même échantillon de composition.

En variante, les première et deuxième régions appartiennent à des échantillons différents.

En variante encore, les première et deuxième régions correspondent à une même région et les éclairages diffus et directionnel sont appliqués alternativement.

L'invention a encore pour objet, selon un autre de ses aspects, un présentoir comportant :

- une source d'éclairage directionnel,
- une source d'éclairage diffus,
- un support pour permettre d'exposer au moins un échantillon de composition à la source d'éclairage diffus et au moins un échantillon de composition à la source d'éclairage directionnel.

Procédé de maquillage

L'invention a encore pour objet un procédé de maquillage des matières kératiniques au moyen d'un produit selon l'invention.

Il peut s'agir du maquillage de la peau, des lèvres ou cheveux.

Le maquillage peut par exemple s'effectuer sans mélange des compositions à l'application.

On peut par exemple déplacer un stick par un mouvement de translation de façon à créer deux traces colorées ayant des couleurs différentes et correspondant chacune au dépôt de la composition de l'un des blocs.

L'application peut encore s'effectuer de manière à provoquer un mélange ou une superposition des compositions des blocs lors de l'application.

Dans une variante où les blocs de composition sont contenus dans une coupelle, chacune des compositions est par exemple prélevée au moyen d'un applicateur ou du doigt.

<u>Exemple proposé</u>

Les teneurs indiquées sont massiques.

<u>Exemple 10 :</u> <u>Rouge à lèvres</u>

Première composition

| | |
|---|---|
| Tri-mellitate de tri-decyle | 11 |
| Lanoline liquide | 10 |
| Malate de d'iso-stearyle | 12 |
| Lanoline acetylée | 10 |
| Triglycerides d'acides laurique/palmitique/cetylique/stearique (50/20/10/10) | 5 |
| Cire microcrystalline (C20-C60) | 3 |
| Lanolate d'isopropyle protégé | 9 |
| Octyl-2-decanol | 15 |
| Phényl trimethylsiloxy trisiloxane (VISCOSITE: 20 CST - PM: 372) | 4 |
| Cire de polyethylene (PM :500) | 8 |
| Pigment interferentiel multicouche * | 10 |
| Pigment à réflexion métallique** | 3 |
| * TIMIRON SILK RED de la société MERCK<br>*** METASHINE ME 2040 PS* de la société NIPPON SHEET GLASS | |

Deuxième composition

| | |
|---|---|
| Tri-mellitate de tri-decyle | 11 |
| Lanoline liquide | 10 |
| Malate de d'iso-stearyle | 12 |
| Lanoline acetylée | 10 |
| Triglycerides d'acides laurique/palmitique/cetylique/stearique (50/20/10/10) | 5 |
| Cire microcrystalline (C20-C60) | 3 |
| Lanolate d'isopropyle protégé | 9 |
| Octyl-2-decanol | 15 |
| Phényl trimethylsiloxy trisiloxane (VISCOSITE: 20 CST - PM: 372) | 4 |
| Cire de polyethylene (PM :500) | 8 |
| Pigment interferentiel multicouche * | 10 |
| Pigment à réflexion métallique** | 3 |
| * TIMIRON SILK BLUE de la société MERCK<br>*** METASHINE ME 2040 PS* de la société NIPPON SHEET GLASS | |

Les deux compositions sont coextrudées pour former deux blocs produisant à l'application des couleurs différentes.

La première composition, rouge, occupe par exemple le centre du stick et la deuxième composition, bleue, sa périphérie, les deux blocs étant concentriques. Du violet peut apparaître à l'application, au centre, délimité par des bordures bleues.

Bien entendu, l'invention n'est pas limitée aux exemples qui viennent d'être donnés. L'expression « comportant un » est synonyme de « comportant au moins un » et « compris entre » s'entend bornes incluses.

**Revendications**

1. Composition cosmétique de couvrance supérieure ou égale à 25, mieux 30, comportant, dans un milieu cosmétiquement acceptable aqueux, au moins un pigment interférentiel multicouche en une teneur conférant à la composition une variation de couleur ΔE à l'application supérieure ou égale à 5.

2. Composition cosmétique de couvrance supérieure ou égale à 25, mieux 30, comportant, dans un milieu cosmétiquement acceptable anhydre, au moins un pigment interférentiel multicouche en une teneur conférant à la composition une variation de couleur ΔE à l'application supérieure ou égale à 5.

3. Composition selon l'une des revendications 1 et 2, la composition étant non pulvérulente et la teneur massique totale en pigment interférentiel multicouche allant de 7 à 20 %.

4. Composition selon la revendication 3, étant liquide (à 25 °C).

5. Composition selon la revendication 3, étant sous forme de stick.

6. Composition selon l'une des revendications 3 à 5, la teneur massique totale en pigment interférentiel multicouche allant de 8 à 15 %.

7. Composition selon l'une quelconque des revendications 1 à 6, comportant une huile, notamment à une teneur massique supérieure ou égale à 30 %.

8. Composition selon l'une quelconque des revendications 1 à 7, comportant une cire, notamment à une teneur massique supérieure ou égale à 10 %.

9. Composition selon l'une quelconque des revendications 1 à 8, comportant une charge, notamment à une teneur massique supérieure ou égale à 10 %.

10. Composition selon l'une des revendications 1 et 2, la composition étant pulvérulente et la teneur massique totale en pigment interférentiel multicouche allant de 40 à 95 %.

11. Composition selon l'une quelconque des revendications 1 à 10, la couleur de la composition dans la masse étant blanche.

12. Composition selon la revendication 14, la composition dans la masse ayant un indice de blancheur supérieur ou égal à 40.

13. Composition cosmétique de couvrance supérieure ou égale à 25 comportant, dans un milieu cosmétiquement acceptable, au moins un pigment interférentiel multicouche non opaque apte à conférer à la composition une couleur blanche dans la masse.

14. Composition cosmétique solide, notamment en stick, comportant, dans un milieu cosmétiquement acceptable, au moins un pigment interférentiel multicouche apte à conférer à la composition une couleur blanche dans la masse et à provoquer après application une variation de couleur ΔE de la composition d'au moins 5.

15. Composition cosmétique selon la revendication 14, la composition étant en stick.

16. Composition selon la revendication 14 ou 15, la composition dans la masse ayant un indice de blancheur supérieur ou égal à 40.

**17.** Composition selon l'une quelconque des revendications 14 à 16, la composition étant anhydre.

**18.** Composition selon l'une quelconque des revendications 14 à 16, la composition étant aqueuse.

**19.** Composition cosmétique comportant, dans un milieu cosmétiquement acceptable, au moins un pigment interférentiel multicouche apte à conférer à la composition une couleur blanche dans la masse, d'indice de blancheur supérieur ou égal à 40, la teneur massique totale en pigment interférentiel multicouche allant de 7 à 20 %, mieux 8 à 15 %, pour une composition non pulvérulente et allant de 40 à 95 %, mieux 40 à 80 %, en masse pour une composition pulvérulente.

**20.** Composition selon l'une quelconque des revendications 1 à 19, la composition ne comportant pas d'autre agent de coloration que le ou les pigments interférentiels multicouche.

**21.** Composition selon l'une quelconque des revendications 1 à 20, le pigment interférentiel multicouche comportant au moins quatre couches.

**22.** Composition selon l'une quelconque des revendications 1 à 21, le pigment interférentiel multicouche comportant un substrat en une matière transparente.

**23.** Composition selon l'une quelconque des revendications 1 à 22, la couleur produite par le pigment interférentiel multicouche à l'application n'étant pas de longueur d'onde dominante comprise entre 580 et 650 nm.

**24.** Composition selon l'une quelconque des revendications 1 à 23, ne comportant qu'un seul pigment interférentiel multicouche.

**25.** Composition selon l'une quelconque des revendications 1 à 23, comportant au moins deux pigments interférentiels multicouche.

**26.** Composition selon la revendication 25, les deux pigments interférentiels multicouche ayant des couches constituées des mêmes matériaux mais au moins une couche d'un pigment ayant une épaisseur différente d'une couche correspondante de l'autre pigment.

**27.** Composition selon l'une des revendications précédentes, le pigment interférentiel multicouche comportant un substrat de silice, mica, verre, alumine ou métal.

**28.** Composition selon la revendication 27, le substrat étant en silice, mica ou verre.

**29.** Composition selon l'une quelconque des revendications 1 à 28, comportant moins de 0,5% en masse de pigments générant une couleur par absorption.

**30.** Composition selon la revendication 29, la composition contenant moins de 0,5% en masse de pigments à base d'oxyde de fer.

**31.** Composition selon l'une quelconque des revendications précédentes, le pigment interférentiel multicouche étant dépourvu de couche colorée.

**32.** Composition selon l'une quelconque des revendications précédentes, le pigment interférentiel ne comportant, sur le substrat, qu'une ou plusieurs couches choisies parmi $TiO_2$, $ZrO_2$, $SnO_2$, $SiO_2$.

**33.** Composition selon l'une quelconque des revendications précédentes, le pigment interférentiel multicouche étant dépourvu de $Fe_2O_3$.

**34.** Composition selon l'une quelconque des revendications 1 à 33, la teneur massique en pigment interférentiel multicouche étant supérieure à 7%.

**35.** Procédé de maquillage des matières kératiniques, comportant l'application sur les matières kératiniques d'une composition selon l'une quelconque des revendications 1 à 34.

**36.** Ensemble de conditionnement comportant :

- une composition telle que définie dans l'une quelconque des revendications 1 à 34,
- un moyen permettant de renseigner l'utilisateur sur la couleur de la composition après l'application sur les matières kératiniques.

**37.** Produit cosmétique solide, notamment en stick, comportant au moins :

- un premier bloc d'une première composition cosmétique contenant, dans un milieu cosmétiquement acceptable, au moins un premier pigment interférentiel multicouche,
- un deuxième bloc d'une deuxième composition cosmétique, contenant dans un milieu cosmétiquement acceptable, au moins un deuxième pigment interférentiel multicouche différent du premier,
l'une au moins des première et deuxième compositions étant apte à présenter une première couleur dans la masse et une deuxième couleur après application sur des matières kératiniques, les première et deuxième couleurs différant d'un écart ΔE supérieur ou égal à 2, mieux 5.

**38.** Produit selon la revendication 37, chacune des première et deuxième compositions étant apte à présenter une première couleur au sein du produit et une deuxième couleur après application sur des matières kératiniques, les première et deuxième couleurs de chaque composition différant d'un écart ΔE supérieur ou égal à 5.

**39.** Produit selon la revendication 37 ou 38, les premier (A) et deuxième (B) blocs s'étendant sur toute la longueur du produit (S).

**40.** Produit selon la revendication 39, chaque bloc (A) ou (B) ayant une section transversale constante sur toute la largeur du produit.

**41.** Produit selon la revendication 39, l'un des blocs au moins présentant une section transversale variable le long de la longueur du produit.

**42.** Produit selon la revendication 37 ou 38, les premier (A) et deuxième (B) blocs étant concentriques.

**43.** Produit selon l'une quelconque des revendications 37 à 41, l'un des blocs présentant une section transversale circulaire.

**44.** Produit selon la revendication 41, la section transversale d'un bloc passant d'une section transversale maximale à une extrémité longitudinale du produit à une section transversale minimale à l'extrémité longitudinale opposée.

**45.** Produit selon l'une quelconque des revendications 37 à 43, l'une au moins des première et deuxième compositions étant anhydre.

**46.** Produit selon l'une quelconque des revendications 37 à 43, l'une au moins des première et deuxième compositions étant aqueuse.

**47.** Produit selon l'une quelconque des revendications 37 à 44, la teneur massique totale en pigment interférentiel multicouche dans l'une au moins des première et deuxième compositions allant de 7 à 20 %.

**48.** Produit selon la revendication 47, la teneur allant de 8 à 15 %.

**49.** Produit selon l'une quelconque des revendications 37 à 48, la couleur de l'une au moins des compositions dans la masse étant blanche.

**50.** Produit selon la revendication 49, la composition dans la masse ayant un indice de blancheur supérieur ou égal à 40.

**51.** Produit selon l'une quelconque des revendications 37 à 50, l'une au moins des compositions ne comportant pas d'autre agent de coloration que le ou les pigments interférentiels multicouche.

**52.** Produit selon l'une quelconque des revendications 37 à 51, le pigment interférentiel multicouche de l'une au moins des compositions comportant au moins quatre couches.

**53.** Produit selon l'une quelconque des revendications 37 à 52, le pigment interférentiel multicouche l'une au moins des compositions comportant un substrat en une matière transparente.

**54.** Produit selon l'une quelconque des revendications 37 à 53, le pigment interférentiel multicouche de l'une au moins des compositions comportant un substrat en silice, mica verre, alumine ou métal.

**55.** Produit selon l'une quelconque des revendications 37 à 54, l'une au moins des première et deuxième compositions ne comportant qu'un seul pigment interférentiel multicouche.

**56.** Produit selon l'une quelconque des revendications 37 à 54, l'une au moins des compositions comportant au moins deux pigments interférentiels multicouche.

**57.** Produit selon la revendication 56, les deux pigments interférentiels multicouche ayant des couches constituées des mêmes matériaux mais au moins une couche d'un pigment ayant une épaisseur différente d'une couche correspondante de l'autre pigment.

**58.** Produit selon l'une quelconque des revendications 37 à 57, les compositions ayant deux pigments interférentiels multicouche respectifs ayant des couches constituées des mêmes matériaux, au moins une couche d'un pigment ayant une épaisseur différente d'une couche correspondante de l'autre pigment, de façon à produire des couleurs différentes.

**59.** Procédé de maquillage des matières kératiniques, comportant l'application sur les matières kératiniques d'au moins les première et deuxième compositions d'un produit selon l'une quelconque des revendications 37 à 58.

**60.** Procédé selon la revendication 59, les première et deuxième compositions étant appliquées simultanément.

**61.** Procédé selon la revendication 59, les compositions étant appliquées de manière à créer des traces de couleurs différentes.

**62.** Dispositif de conditionnement et de distribution comportant :

- un produit tel que défini dans l'une quelconque des revendications 37 à 58,
- un corps creux dans lequel le produit est logé au moins partiellement.

A

S

Fig.1

B

A

S

Fig.3

B

Fig.6

B

A

A

B

S

C

Fig.2

M

A   B   E

Fig.4

A   B

Fig.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1172091 A

**Littérature non-brevet citée dans la description**

- International Cosmetic Ingredient Dictionnary and Handbook. The Cosmetic, Toiletry, and Fragrance Association, 1997, 371-386524-528